(19) Europäisches Patentamt
European Patent Office
Office européen des brevets

(11) **EP 2 607 897 B1**

(12) **EUROPÄISCHE PATENTSCHRIFT**

(45) Veröffentlichungstag und Bekanntmachung des Hinweises auf die Patenterteilung:
**07.11.2018 Patentblatt 2018/45**

(51) Int Cl.:
***G01N 33/02*** *(2006.01)*

(21) Anmeldenummer: **12008377.9**

(22) Anmeldetag: **17.12.2012**

(54) **Verfahren zur Bestimmung eines Kennwerts für die Frische und/oder den Frischeverlust einer Lebensmittel-Probe**

Method for determining a value for the freshness and/or freshness loss of a food sample

Procédé de détermination d'une caractéristique pour la fraîcheur ou la perte de fraîcheur d'un échantillon de nourriture

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.12.2011 DE 102011122234**
**14.05.2012 EP 12003780**

(43) Veröffentlichungstag der Anmeldung:
**26.06.2013 Patentblatt 2013/26**

(73) Patentinhaber: **QFood GmbH**
**79194 Gundelfingen (DE)**

(72) Erfinder:
• **Seidel, Robert**
**79102 Freiburg (DE)**
• **Klapproth, Holger**
**79108 Freiburg (DE)**
• **Bednar, Sonja**
**79194 Gundelfingen (DE)**

(74) Vertreter: **Huwer, Andreas et al**
**Huwer & Partner**
**Patent- und Rechtsanwälte PartG mbB**
**Schwaighofstrasse 1**
**79100 Freiburg (DE)**

(56) Entgegenhaltungen:
• **TOKUNAGA T: "Studies on the quality evaluation of canned marine products. I. Determination of the ratio of dimethylamine to trimethylamine in canned albacore. (translated)", NIPPON SUISAN GAKKAISHI - BULLETIN OF THE JAPANESE SOCIETY OFSCIENTIFIC FISHERIES, NIPPON SUISAN GAKKAI, TOKYO, JP, Bd. 41, Nr. 5, 1. Januar 1975 (1975-01-01) , Seiten 547-553, XP008153847, ISSN: 0021-5392**
• **OLGA SOLOMON ET AL: "Effect of oxygen and fluorescent light on the quality of orange juice during storage at 8°C", FOOD CHEMISTRY, Bd. 53, Nr. 4, 1. Januar 1995 (1995-01-01) , Seiten 363-368, XP055033308, ISSN: 0308-8146, DOI: 10.1016/0308-8146(95)99828-N**
• **I. T. AGAR ET AL: "Postharvest CO2 and Ethylene Production and Quality Maintenance of Fresh-Cut Kiwifruit Slices", JOURNAL OF FOOD SCIENCE, Bd. 64, Nr. 3, 1. Mai 1999 (1999-05-01), Seiten 433-440, XP055033181, ISSN: 0022-1147, DOI: 10.1111/j.1365-2621.1999.tb15058.x**
• **KERRY J P ET AL: "Past, current and potential utilisation of active and intelligent packaging systems for meat and muscle-based products: A review", MEAT SCIENCE, ELSEVIER SCIENCE, GB, Bd. 74, Nr. 1, 1. September 2006 (2006-09-01), Seiten 113-130, XP027989844, ISSN: 0309-1740 [gefunden am 2006-09-01]**

**EP 2 607 897 B1**

**Beschreibung**

Die Erfindung betrifft ein Verfahren nach dem Oberbegriff von Anspruch 1.

Inhaltsstoffe von Lebensmitteln wie Vitamine, Nukleinsäuren, Eiweiße und Aminosäuren u. a. unterliegen einer natürlichen Alterung wie beispielsweise Oxidation oder enzymatische Umsetzung als Funktion der Zeit, abhängig z.B. von Parametern wie Temperatur, Lichteinwirkung, pH-Wert oder dem Aussetzen bestimmter Gase oder Schwermetallionen. Wichtige Wirkungen oder gewünschte Inhaltsstoffe sind nach Überschreiten einer zu definierenden Zeitdauer nicht mehr in ausreichender Menge verfügbar oder zerfallen sogar in schädliche Nebenprodukte.

Ein Verfahren zur Bestimmung eines Kennwerts für die Frische einer Lebensmittel-Probe, die das Ausgangsgangsprodukt Ascorbinsäure enthält, ist aus Olga Solomon et al: "Effect of oxygen and fluorescent light on the quality of orange juice during storage at 8°C", Food Chemistry, Bd. 53, Nr. 4, 1. Januar 1995 (1995-01-01), Seiten 363-368, ISSN: 0308-8146, DOI: 10.1016/0308-8146(95)99828-N bekannt. Beim Auftreten einer ersten Frischeverlustart wird die Ascorbinsäure zersetzt, wobei ein erster Metabolit, nämlich DHA gebildet wird. Für die Konzentration der Ascorbinsäure wird ein erster Ausgangsprodukt-Konzentrationswert, nämlich der gesamte Vitamin C Gehalt, und für die Konzentration des DHA ein erster Metabolitkonzentrationswert gemessen. Die beiden Messungen erfolgen gleichzeitig. Der Kennwert für die Frische der Lebensmittelprobe wird in Abhängigkeit vom Verhältnis DHA/(gesamter Vitamin C Gehalt) bestimmt. Zusätzlich wird die HMF-Konzentration gemessen. Es zeigte sich, dass die HMF-Konzentration beim Auftreten eines Frischeverlusts keiner wesentlichen Veränderung unterliegt.

Bei einem anderen, aus dem Stand der Technik bekannten Verfahren wird ebenfalls das Ausgangsprodukt Ascorbinsäure (Vitamin C) verwendet. Um Ascorbinsäure quantitativ nachzuweisen, gibt es zahlreiche kolorimetrische Methoden. Normalerweise verwendet man 2,4-Dinitrophenylhydrazin. Ascorbinsäure reagiert mit diesem zu einem Hydrazon, dessen Absorption gemessen werden kann. Darüber hinaus kann 2,2'-Bipyridin zum colorimetrischen Nachweis dienen. Hierbei macht man sich die Reduktionskraft der Ascorbinsäure zunutze, die Fe(III) zu Fe(II) reduziert. Fe(II) bildet dann mit 2,2'-Bipyridin einen farbigen Komplex Es sind auch einige fluorometrische Nachweismethoden bekannt.

Ascorbinsäure lässt sich auch durch Titration mit Tillmans' Reagenz (2,6-Dichlorphenolindophenol, abgekürzt DCPIP) nachweisen, bei der das Reagenz durch die Ascorbinsäure zu einer Leukoverbindung reduziert wird. Dabei ist ein Farbumschlag von tiefblau zu farblos zu beobachten. Diese Methode eignet sich für eine schnelle Bestimmung, die aber an die Genauigkeit oben genannter Wege nicht heranreicht. [Quelle: Wikipedia]

Ascorbinsäure kann auch spezifisch mittels Oxidation durch das Enzym Ascorbinsäure-Oxidase nachgewiesen werden, wobei die Änderung der Lichtabsorption bei einer Wellenlänge von 245 nm gemessen wird.

Für Fisch, der kein Vitamin C enthält, muss ein anderes Ausgangsgangsprodukt als Frischemarker verwendet werden. Ein idealer Parameter für die Frischemessung in Salzwasserfischen ist Trimethylamin (TMA). TMA entsteht bei dem bakteriellen Abbau von Trimethyaminoxid) und ist für den "fischigen" Geruch verantwortlich. TMAO kann aber auch enzymatisch in Dimethylamin (DMA) und Formaldehyd gespalten werden, wobei das Formaldehyd mit dem Gewebe reagiert und deswegen schlecht bestimmbar ist. Trimethyamin und Dimethlyamin werden normalerweise per HPLC (Hochleistungsflüssigkeitschromatographie) bestimmt. Da jedoch diverse Fische verschiedene Ausgangskonzentrationen an TMAO aufweisen und sowohl der TMA als auch der DMA Stoffwechselweg eingeschlagen werden kann ist es notwendig eine genauere Methode zum Nachweis zu haben.

In Fleisch und Süßwasserfischen können verschiedene Ausgangsgangsprodukte als Frischemarker verwendet werden. Einer der einfachstem Frischemarker ist Histamin, das aus Histidin durch Decarboxylierung entsteht. Weitere biogene Amine, die als Frischemarker dienen können, sind beispielsweise aber nicht ausschließlich: Putrescin, Cadaverin und Tyramin. Putrescein entsteht durch Zersetzung von Ornithin, Cadaverin entsteht durch die Zersetzung von Lysin, Tyramin und durch die Zersetzung von Tyrosin. Da alle diese Frischemarker auf einen bakteriellen Zersetzungsprozess hinweisen, sind diese Stoffe gut geeignet die Lagerungstemperatur und die hygienischen Bedingungen bei der Verarbeitung von Fleisch und Fisch abzubilden. Auch in diesem Fall ist es sinnvoll den Gehalt der Ausgangssubstanz mit zu bestimmen, um so eine Information über den Grad der Zersetzung zu bekommen. In Histidin armem Gewebe ist es nicht verwunderlich, wenn der Gehalt an Histamin gering ist.

Bei einem aus Toshio Tokunaga: "Studies on the quality evaluation of canned marine products - I. Determination of the ratio of dimethylamine to trimethylamine in canned albacore" (translated), Nippon Suisan Gakkaishi - Bulletin Of The Japanes Society Of Scientific Fisheries, Nippon Suisan Gakkai, Tokyo, JP, Bd. 41, Nr. 5, 1. Januar 1975 (1975-01-01), Seiten 547-553, ISSN: 0021-5392 bekannten Verfahren werden zum Bestimmen eines Kennwerts für die Frische einer Fischprobe die Konzentration von DMA-N und TMA-N gemessen und zueinander ins Verhältnis gesetzt.

Abbauprodukte von Nukleinsäuren

Der Abbau von Nukleinsäuren in Fleisch und Fisch ist ein guter Marker für die Frische, da dort die Nukleinsäuren über autolytische Prozesse und bakterielle Prozesse abgebaut werden. Stand der Technik ist hier der Nachweis über HPLC, der aber eine aufwendige Aufreinigung erfordert (Veciana-Nogues 1997, Determination of ATP related compounds in fresh and canned tuna fish by HPLC, Food Chemistry 59(3)). Andere Testsysteme erlauben nur die isolierte Bestimmung von individuellen Analyten (siehe Tabelle 1).

Der Abbau verläuft dabei von den energiereichen Triphopsphaten zu den energieärmeren Nukleotiden bzw den abgespaltenen Basen:

ATP (Adenosintriphopshat) - ADP (Adenosindiphosphat) - AMP (Adenosinmonophosphat) - IMP (Inosinmonophosphat) - Ino (Inosin) - Hx (Hypoxanthin)

Table 1: Frischetest unter Verwendung der Enzym-Technologie

| Analyt (e) | Prinzip | Vorteil | Nachteil | Referenz |
|---|---|---|---|---|
| Hx | auf einem Teststreifen immobilisierte Enzyme (xanthine oxidase, X0) | schnell, außerhalb eines Labors einfach zu verwenden | halbquantitativ, nur geeignet für Messung von Hx (Folge des Verderbens) | Jahns et al. (1976) |
| Hx, Ino | Teststreifen mit immobilisierten Enzymen | schnell, außerhalb eines Labors einfach zu verwenden | halbquantitativ, geringe Reproduzierbarkeit, begrenzt auf Hx und Ino (Folge des Verderbens) | Ehira et al. (1986) |
| IMP, Ino, Hx | enzymbeschichtete Sauerstoffelektrode | schnell, genau | komplizierter und zeitaufwändiger als Teststreifen-Technologie | Karube et al. (1984) |
| K-index | Versuch mit gekoppelten Emzymen "KV-101 Freshness Meter" | schnell, Ergebnisse vergleichbar mit HPLC | es müssen Enzyme und Reagenzien gekauft werden, Kosten? | handelsübliche Form, Orienta Electric, Niiza Saitama 352, Japan |
| K-index | enzymbeschichtete Sauerstoffelektrode "Microfresh" | schnell, Ergebnisse vergleichbar mit HPLC | Kosten? | handelsübliche Form, Pegasus Instruments, Agincourt, ON, Canada |
| Quelle: FAO FISHERIES TECHNICAL PAPER - 348 FOOD AND AGRICULTURE ORGANIZATION OF THE UNITED NATIONS, H. H. Huss Technological Laboratory, Ministry of Agriculture and Fisheries Denmark, FOOD AND AGRICULTURE ORGANIZATION OF THE UNITED NATIONS Rome, 1995 | | | | |

Im Stand der Technik werden einzelne Substanzen oder auch Gruppen verwandter Substanzen einzeln oder sequentiell gemessen. Jedoch muss für jede Substanz / Substanzgruppe eine eigene Analyse durchgeführt werden. Verwendete Verfahren dafür sind enzymatische Tests (ELISA), chromatographische Verfahren mit optischer Detektion (HPLC) oder photometrische Verfahren. Alle diese Verfahren haben den Nachteil, dass diese entweder nur einen Parameter erfassen können oder eine aufwendige Probenaufbereitung erfordern. Bei der HPLC Messung, bei der zwar für ein Ausgangsgangsprodukt mehrere ähnliche Zersetzungsprodukte gemessen werden, können, werden die Zersetzungsprodukte per Chromatographie getrennt und es muss im Vorfeld eine aufwendige Probenvorbereitung erfolgen. Gleichzeitig liegt das Problem vor, dass ein Frischeverlust auf verschiedenen Wegen stattfinden kann. So kann durch eine Wärmebelastung sowohl eine Autolyse als auch eine bakterielle Zersetzung stattfinden. Weitere Ursachen für einen Frischeverlust sind z.B. Oxidation und eine Zerstörung der Zellmembranen durch Einfrieren und wieder Auftauen.

In der vorliegenden Patentanmeldung wird unter einem Frischeverlust eine Minderung (Änderung) des Qualität eines Lebensmittel durch Alterungsvorgänge verstanden Diese reduzieren z.B. den Nährwert, ändern den Geschmack und/oder Geruch, die optische und/oder haptischen Eigenschaften des Lebensmittels. Oftmals sind diese Vorgänge durch chemische / biochemische Reaktionen bedingt z.B. Wasserverlust, Hydrolyse, Abbau von Nährstoffen, stoffliche Umsetzung durch Mikroorganismen.

Aufgabe der Erfindung ist es, ein Verfahren der eingangs genannten Art zur Verfügung zu stellen, das es ermöglicht, einen Kennwert für die Frische und/oder den Frischeverlust eines Lebensmittels schnell und einfach, aber dennoch möglichst exakt zu bestimmen.

Erfindungsgemäß wird diese Aufgabe durch ein Verfahren mit den Merkmalen des Anspruchs 1 gelöst. Dabei werden mehrere lebensmittelspezifische Parameter, nämlich die Konzentrationen von mindestens zwei Ausgangsprodukten mit einer beschränkten Halbwertszeit und von mindestens zwei Metaboliten in einer vorzugsweise wasserhaltigen Lebenmittelprobe, gleichzeitig ermittelt, um so die Frische möglichst exakt zu bestimmen. Durch die gleichzeitige Erfassung der Konzentrationen, welche die verschiedenen Aspekte der Frische darstellen, wird der Aufwand der Messung möglichst gering gehalten. In vorteilhafter Weise werden außerdem die beim Stand der Technik auftretenden Probleme, wie z.B. aufwendige Probenvorbereitung bzw. multiple Bestimmungen, um die Analyte zu ermitteln, sowie eine Abnahme der Frische der Lebensmittelprobe zwischen zeitlich voneinander beabstandeten Konzentrationsmessungen, vermieden. Das Verfahren kann beispielsweise zum Zwecke des Verbraucherschutzes Anwendung finden, um auf der Verpackung von Lebensmitteln angebrachte Haltbarkeitsangaben zu überprüfen. Auch für die Qualitätskontrolle ist der erfindungsgemäße Frischetest ein wichtiges Hilfsmittel.

Der mindestens eine Interaktionswert kann experimentell anhand von Versuchsreihen ermittelt werden. Er kann aber auch aufgrund von Erfahrungswerten bereitgestellt werden. Der mindestens eine Interaktionswert ist ein Interaktionsfaktor, der für den Fall, dass die miteinander interagierenden Frischeverlustarten bei der Lebensmittel-Probe vorliegen, bei der Ermittlung des Kennwert für die Frische oder den Frischeverlust des Lebensmittels mit einem für eine dieser Frischeverlustarten ermittelten Verhältnisistwert multipliziert wird.

Erfindungsgemäß wird die Interaktion zwischen den Frischeverlustarten bzw. der Einfluss, den die Frische einer ersten Frischeart auf die Frische einer zweiten Frischeart hat, berücksichtigt, wodurch die Haltbarkeitsdauer der Lebensmittel-Probe mit großer Präzision bestimmt werden kann. Der Sollwert für die Haltbarkeitsdauer kann der für das betreffende Lebensmittel unter Berücksichtigung vorgeschriebener oder vorgegebener Umgebungsbedingungen typischen Haltbarkeitsdauer entsprechen. Die Umgebungsbedingungen können insbesondere die Lagertemperatur und/oder die das Lebensmittel umgebende Atmosphäre (z.B. Luft, Schutzgas), die Luftfeuchtigkeit und/oder die Keimbelastung in der Umgebung des Lebensmittels umfassen. Der Sollwert für die Haltbarkeitsdauer kann zum Beispiel ein Erfahrungswert sein und/oder aufgrund von experimentellen Untersuchungen bereitgestellt werden. Das vorstehend genannte Verfahren kommt bevorzugt nur dann zur Anwendung, wenn keiner der ermittelten Haltbarkeitsdauerwerte kleiner ist als der Sollwert für die Haltbarkeitsdauer. Sollte dies nicht der Fall sein, wird davon ausgegangen, dass das untersuchte Lebensmittel nicht mehr verkehrsfähig ist. Bei Bedarf können die Referenzwertkombinationen auch in Form einer Kennlinie und/oder einer Berechnungsvorschrift bereitgestellt werden.

Bei einer vorteilhaften Ausgestaltung des Verfahrens-enthält die Lebensmittel-Probe mindestens ein drittes Ausgangsgangsprodukt, das beim Auftreten einer dritten Frischeverlustart unter Bildung eines dritten Metabolits zersetzt wird, wobei für die Konzentration des dritten Ausgangsprodukts ein dritter Ausgangsprodukt-Konzentrationsistwert und für die Konzentration des dritten Metaboliten ein dritter Metabolit-Konzentrationsistwert gemessen wird, und wobei der Kennwert für die Frische der Lebensmittelprobe in Abhängigkeit vom Verhältnis des dritten Ausgangsprodukt-Konzentrationsistwerts zum dritten Metabolit-Konzentrationsistwert ermittelt wird. Dadurch kann der Kennwert für die Frische bzw. den Frischeverlust noch exakter ermittelt werden.

Bei einer bevorzugten Ausführungsform der Erfindung werden für die dritte Frischeverlustart dritte Referenzwertkombinationen bereit gestellt, die jeweils einen vom Verhältnis zwischen dem Ausgangsprodukt-Konzentrationswert für das dritte Ausgangsgangsprodukt und dem Metabolit-Konzentrationswert für den dritten Metabolit abhängigen dritten Verhältnisreferenzwert sowie einen diesem zugeordneten dritten Haltbarkeitsdauerreferenzwert aufweisen, wobei ein vom Verhältnis zwischen dem dritten Ausgangsprodukt-Konzentrationsistwert und dem dritten Metabolit-Konzentrationsistwert abhängiger dritter Verhältnisistwert gebildet wird, wobei in Abhängigkeit von den dritten Referenzwertkombinationen und dem dritten Verhältnisistwert für die dritte Frischeverlustart ein dritter Haltbarkeitsdauerwert ermittelt wird, wobei für jede Frischeverlustart, für die nicht der größte Differenzwert ermittelt wurde, jeweils ein Interaktionswert für den Einfluss, den der Frischeverlust der Frischeverlustart, für welche der größte Differenzwert ermittelt wurde, auf den Frischeverlust der zuerst genannten Frischeverlustart hat, bereitgestellt wird, und wobei der Kennwert für die Frische aus dem Sollwert für die Haltbarkeitsdauer, den Differenzwerten und den Interaktionswerten gebildet wird. Bei dem erfindungsgemäßen Verfahren kann also auch der Einfluss von mehr als zwei Frischeverlustarten auf die Frische bzw. die Resthaltbarkeit des Lebensmittels berücksichtigt werden.

Erfindungsgemäß wird der Kennwert für die Frische in der Weise gebildet, indem alle Differenzwerte, mit Ausnahme des größten Differenzwerts, jeweils mit einem ihnen zugeordneten Interaktionswert multipliziert werden, und das Ergebnis dieser Multiplikation oder die Ergebnisse dieser Multiplikationen sowie der größte Differenzwert vom Sollwert für die Haltbarkeitsdauer subtrahiert werden.

Bei einer Weiterbildung der Erfindung wird der Kennwert für die Frische in Abhängigkeit von den Frischeverlustarten ermittelt. In dem Kennwert sind also auch Informationen über die Art des Frischeverlusts enthalten, so dass anhand des Kennwerts die Ursache des Frischeverlusts ermittelt werden kann. Bevorzugt wird in dem Kennwert der Frischeverlust für die einzelnen Frischeverlustarten getrennt quantitativ erfasst, so dass beispielsweise ersichtlich ist, durch welche Frischeverlustart (wie z.B. Temperatureinwirkung bzw. Unterbrechung der Kühlkette, Einwirkung von Bakterien, Lichteinwirkung, Oxidation etc.) die Frische wie stark abgenommen hat.

Vorteilhaft ist, wenn die Ausgangsprodukt-Konzentrationsistwerte und die Metabolit-Konzentrationsistwerte mittels Fourier-Transformations-Infrarotspektroskopie (FTIR) gemessen werden. Die Messung kann dann im Zuge einer Routine-Qualitätsmessung von Facharbeitern durchgeführt werden und es wird kein spezielles Labor benötigt.

Bei einer bevorzugten Ausgestaltung der Erfindung wird gleichzeitig zur Messung der Ausgangsprodukt-Konzentrationsistwerte und zur Messung der Metabolit-Konzentrationsistwerte der Nährwert der Probe bestimmt. Da Frische bei Lebensmitteln aber neben einer Altersangabe auch ein Maß für den Nährwert und die gesundheitliche Unbedenklichkeit ist, ist diese Ausgestaltung des Verfahrens vorteilhaft. Bei Bedarf kann zusätzlich zum Nährwert auch der Vitamingehalt ermittelt werden.

Vorteilhaft ist, wenn zum Ermitteln der Metabolit-Konzentrationsistwerte und der Ausgangsprodukt-Konzentrationsistwerte Messwerte erfasst werden, wenn die Messwerte matrixspezifisch korrigiert werden, und wenn die Konzentrationsistwerte mit Hilfe der korrigierten Messwerte ermittelt werden. Dabei wird davon ausgegangen, dass sich in einem Lebensmittel (der Matrix) die spezifischen infrarot-Absorptionsbanden einer Substanz durch die Interaktion mit den anderen Bestandteilen des Lebensmittels verändern. Ändern sie diese (z.B. wenn Milch sauer wird) dann können diese Interaktionen das IR-Spektrum der zu analysierenden Substanz ändern. Diese Effekte werden als Matrixeffekte bezeichnet. Bei der matrixspezifisch Korrektur werden diese Änderungen mit entsprechenden mathematischen Verfahren kompensiert, z.B. durch Verwendung einer Look Up Table für die pH-Wert bedingten Veränderungen. Durch die matrixspezifische Korrektur kann Hintergrundrauschen kompensiert werden. Somit kann der Kennwert für die Frische und/oder den Frischeverlust noch genauer ermittelt werden.

Bevorzugterweise wird eine Vielzahl von Parametern für mehrere verschiede Arten der Alterung (thermisch, bakteriell, autolytisch, chemisch) erfasst und ausgewertet. Bei der Messung solcher Parameter mittels FTIR besteht das Problem, dass Wasser in einem großen Bereich des Infrarot-Spektrums eine starke Absorbtion aufweist. Dadurch ist es extrem schwierig von gering konzentrierten Substanzen ein Spektrum in komplexen Matrices wie z.B. Lebensmitteln aufzunehmen. Es zeigte sich in vielen Messungen, dass durch die Alterung der Lebensmittelprobe die Messergebnisse verändert werden und insbesondere die spektrale Signatur definierter Inhaltsstoffe verändert wird. Dadurch entsteht die Notwendigkeit einer altersgemäßen Korrektur der Proben. Werte, die dabei eine Rolle spielen sind z.B. freie Säuren und Basen, die an Analyte binden können und so deren spektrale Eigenschaften verändern. Überraschenderweise zeigte es sich, dass sich die modifizierten Spektren in diesen Lebensmitteln mathematisch so kompensieren lassen, dass eine Quantifizierung weiterhin möglich ist, wobei die absolute Größe der Änderung des Spektrums ebenfalls als Maß für die Veränderung (= Frischeverlust) verwendet werden kann. Insbesondere lässt sich durch Messung in von extrem dünnschichtigen Flüssigkeitsfilmen (2-50 $\mu$m, insbesondere 10-30 $\mu$m) und die alterungsgemäße Korrektur der Spektren die Nachweisgrenze für quantiatative Infrarot-Spektroskopie auf unter 50 $\mu$M reduzieren. Die Restmenge des Stoffes und die Menge der Zerfallsprodukte sind ein Maß für die Ausgangskonzentration in dem Lebensmittel. Das Verhältnis Restmenge zu Ausgangsmenge ergibt den Kennwert für die Frische des Lebensmittels. Die Art der Zerfallsstoffe erlaubt einen Rückschluss auf die Weise in der das Lebensmittel gealtert ist, z.B. Hitzeeinwirkung, Oxidation, bakterielle Zersetzung.

Das erfindungsgemäße Verfahren ermittelt die Frische von Lebensmitteln anhand von Spektren der Flüssigkeiten der Nahrungsmittel, z.B. von Saft, indem ein spezifischer Inhaltstoff im Verhältnis zu seinen Verfallsprodukten betrachtet wird. Dies geschieht erfindungsgemäß bevorzugt mit IR Spektroskopie, ist aber ebenso mit anderen spektroskopischen oder auch chromatographischen Verfahren möglich. Das erfindungsgemäße Verfahren funktioniert bei allen Stoffen oder Ausgangsgangsprodukten, die eine Verfallssensitivität aufweisen, insbesondere durch Wärme, Licht, pH-Wert, Enzyme, Mikroorganismen oder insbesondere katalytisch wirksame Substanzen, und die ein oder mehrere stabile Verfallsprodukte oder Metaboliten haben. Da die Metabolite je nach Verfallsursache variieren, können auch Aussagen getroffen werden, in welcher Weise das Produkt beansprucht worden ist, z.B. zu viel Wärme oder Enzyme oder Ähnliches.

In einer bevorzugten Ausführungsform der Erfindung wird die Bestimmung des Vitamin C Gehaltes und der Gehalte der Zerfallsprodukte mit Hilfe der Infrarotspektroskopie durchgeführt. Vitamin C (Ascorbinsäure) wird reversibel zu Dehydroascorbinsäure (DHA) oxidiert und irreversibel zu Diketogulonsäure (DKG) und Oxalsäure zersetzt. Unter Hitze wird auch Hydoxymethylfurfural (HMF) gebildet, was einen braunen Farbstoff bildet. Es wird eine flüssige Lebensmittelprobe in einer Microflusszelle (Dicke ca 2 $\mu$m bis 50 $\mu$m, bevorzugt 5-15 $\mu$m) im mittleren Infrarotbereich vermessen und die Zerfallsprodukte und das Vitamin C werden aufgrund Ihrer spezifischen Spektren identifiziert. Solche Flusszellen sind z.B. von der Firma Bruker kommerziell erhältlich. Dazu ist vor allem die "Fingerprint" Region (ca. 2 $\mu$m - 40 $\mu$m) der Infrarotabsorption nützlich. Auf diese Weise kann mit einer Messung und der anschließenden spektralen Analyse die Menge der relevanten Zerfallsprodukte schnell gemessen werden. Insbesondere wird dabei der Gehalt der Probe an Vitamin C, DHA, DKG und HMF gemessen. Die Summe aus Vitamin C und DHA gibt dabei den effektiven Gehalt an Vitamin C für die Ernährung an, wohingegen das Verhältnis vom Vitamin C zur Summe der Zerfallsprodukte ein inverses Maß für die Frische darstellt.

In einer weiteren Ausführungsform der Erfindung werden andere Zerfallsprozesse in den Lebensmitteln analysiert, wobei zur Bestimmung der Frische immer ein Verhältnis Metabolit (Edukt) in Relation zum Ausgangsgangsprodukt gebildet wird. Als Beispiel sei hier HMF in Honig genannt. Eine geringe Menge an HMF im Honig ist ein Indikator für dessen Frische und Naturbelassenheit. Ein hoher HMF-Wert weist auf länger anhaltende Erwärmung oder Lagerung hin. Wenn Honig erhitzt wird, bildet sich aus Fruchtzucker HMF. Der HMF-Gehalt in frisch geschleudertem Honig ist sehr gering und steigt bei korrekter Lagerung, je nach pH-Wert und Lagertemperatur um ca. 2-3 mg/kg pro Jahr an. Lagerung bei Zimmertemperatur (21 °C) kann den HMF-Gehalt in einem Jahr bereits auf 20 mg/kg erhöhen. Die EU hat einen HMF-Grenzwert von maximal 40 mg/kg für Honig, der unter europäischen Bedingungen produziert wurde, festgelegt. Einige nationale Imkerverbände fordern sogar noch niedrigere Werte, z. B. erlaubt der Deutsche Imkerbund höchstens 15 mg/kg für sein Gütesiegel "Echter Deutscher Honig".

Im Stand der Technik wird HMF im Honig meistens mit HPLC- oder dem sogenannten Winkler-Verfahren nachgewiesen. Seit 2009 ist ein Schnelltest von der Merck KGaA zur Bestimmung von HMF erhältlich. Bei dem "Reflectoquant® HMF" genannten Test wird eine geringe Menge Honig im Verhältnis 1:4 mit destilliertem Wasser verdünnt, ein Teststreifen in die Probe getaucht und dann in einem RQflex-Reflektometer gemessen. Alle diese Tests haben die Einschränkung, dass immer nur eine Art von Parameter bzw eine Art von Alterung gemessen wird.

Das erfindungsgemäße Verfahren ist nicht auf bestimmte Stoffe beschränkt, sondern es eignen sich alle Substanzen, die unter bestimmten Bedingungen zu definierten Zerfallsprodukten umgewandelt werden. Solche Substanzen sind z.B. (aber nicht ausschließlich): Thiamin, Pyrodoxin, Cyancobolamin, Phenole, Indol-Essigsäure (Abbau zu 2-Acetaminophenon), Apfelsäure (Abbau der Apfelsäure durch malolaktische Gärung zu Essigsäure), Mannit (enzymatische Umwandlung von Fructose zu Mannit), oxidierbare Fettsäuren ("Ranzigkeit").

Das Verfahren ist besonders geeignet für flüssige Lebensmittel wie z.B. Wein, Bier, Saft, aber auch für andere Lebensmittel, sofern die zu untersuchenden Inhaltsstoffe daraus extrahiert werden können. Dies kann z.B. durch homogenisieren von Fleisch mit einer Pufferlösung oder auch durch Extraktion mit organischen Lösungsmitteln geschehen. Da in dem Verfahren ein Verhältnis aus Ausgangsgangsprodukt zu Metabolit gebildet wird, ist vor allem bei Extrakten die Effizienz nicht von ausschlaggebender Wichtigkeit, da durch die Verrechnung von Ausgangsgangsprodukt zu Metabolit(en) eine dimensionslose Einheit entsteht. Ist das Lebensmittel fest oder schwer extrahierbar, so kann in einer weiteren bevorzugten Ausführungsform der Erfindung die Bestimmung der Paramter mittels ATR-IR Spektroskopie durchgeführt werden. Dazu wird z.B. ein ATR-Kristall auf die Probe gedrückt um dann im Bereich der evaneszenten Wellen die Absorption im IR (MIR) Bereich zu messen. Entsprechende ATR Sonden gibt es kommerziell zu kaufen.

In einer besonders bevorzugten Ausführungsform ist nicht nur das Spektrum der Reinsubstanzen bekannt und auch das Spektrum der gesuchten Substanzen in der relevanten Matrix (Vitamin C in Apfelsaft), sondern auch das Spektrum des entsprechend gealterten Lebensmittels. Die alterungstypischen spektralen Änderungen können dann z.B. mittels einer sog. Hauptkomponentenanalyse (PCA) ermittelt werden und als zusätzliches oder auch alleiniges Identifikationskriterium für das Alter der Lebensmittelprobe dienen. In diesem Fall sind Faktoren wie die einzelnen Substanzen (z.B. Vitamin C, DHA...) in den jeweiligen Spektren mit abgebildet.

In einer weiteren bevorzugten Ausführungsform werden zudem die Faktoren, die den Nährwert des Lebensmittels ausmachen, in der gleichen Messung mitbestimmt. Dies ist möglich, da diese Substanzen alle einen Fingerabdruck im IR-Bereich haben (Signatur), und so mit der gleichen Messung (d.h. gleichzeitig) mit erfasst werden können. Die erfassten Werte müssen dann noch matrixspezifisch ausgewertet werden, da die Matrix (d.h. das Lebensmittel) einen deutlichen Einfluss auf die IR-spezifische Signatur der Einzelkomponenten hat. Faktoren, die den Nährwert ausmachen, sind unter anderem Makronährstoffe und Mikronährstoffe. Diese Substanzen können mittels FT-IR exakt bestimmt werden.

Die Erfindung betrifft also ein Verfahren zur Bestimmung der Frische einer Probe entsprechend dem unabhängigen Anspruch 1.

Die Bestimmung der Metaboliten und deren Ausgangsstoffe kann gleichzeitig stattfinden, vorzugsweise mittels FT-IR.

Nachfolgend wird die Erfindung anhand von Ausführungsbeispielen näher beschrieben.

Es wird Apfelsaft bereitgestellt, bei dem durch Einwirkung von Wärme ein erster, thermischer Frischeverlust und durch Einwirkung von Bakterien ein zweiter, bakterieller Frischeverlust aufgetreten ist. Von dem zu prüfenden Apfelsaft wird eine Probe von 10 ml zur Analyse entnommen. Davon werden 1 ml durch einen Filter der Porengröße 0,4 $\mu$m filtriert. Anschließend werden davon ca. 150 $\mu$l in eine FTIR Mikro-Flusszelle injiziert und mittels FTIR vermessen mittels Licht der Wellenlänge 1-11 $\mu$m. Aus dem komplexen Spektrum werden dann mit Hilfe einer auf einem Computer laufenden Analysesoftware die Konzentrationsistwerte an Vitamin C und DHA (Dehydroascorbinsäure) als Zerfallsprodukt von Vitamin C sowie die Konzentrationsistwerte von Äpfelsäure und Milchsäure bestimmt. Dabei werden folgende Verfahrensschritte durchgeführt:

1. Bestimmen eines ersten Ausgangsprodukt-Konzentrationsistwerts für Vitamin C und eines zweiten Ausgangsprodukt-Konzentrationsistwerts für Äpfelsäure, jeweils unter Verwendung von matrixspezifischen Korrekturen

2. Bestimmen eines ersten Metabolit-Konzentrationsistwerts für DHA und eines zweiten Metabolit-Konzentrationsistwerts für Milchsäure, jeweils unter Verwendung von matrixspezifischen Korrekturen.

3. Bilden eines ersten Verhältniswertes aus dem ersten Ausgangsprodukt-Konzentrationsistwert und dem ersten Metabolit-Konzentrationsistwert.

4. Bilden eines zweiten Verhältniswertes aus dem zweiten Ausgangsprodukt-Konzentrationsistwert und dem zweiten Metabolit-Konzentrationsistwert.

5. Bereitstellen einer Mehrzahl von ersten Referenzwertkombinationen für den thermischen Frischeverlust, die jeweils einen Verhältniswert für das Verhältnis zwischen einem Ausgangsprodukt-Konzentrationswert für Vitamin C und einem Metabolit-Konzentrationswert für DHA sowie einen diesem Verhältniswert zugeordneten ersten Haltbarkeitsdauerwert aufweisen.

6. Bereitstellen einer Mehrzahl von zweiten Referenzwertkombinationen für den bakteriellen Frischeverlust, die jeweils einen Verhältniswert für das Verhältnis zwischen einem Ausgangsprodukt-Konzentrationswert für Äpfelsäure und einem Metabolit-Konzentrationswert für Milchsäure sowie einen diesem Verhältniswert zugeordneten zweiten Haltbarkeitsdauerwert aufweisen.

7. Bewertung der Frische anhand der ersten und zweiten Verhältniswerte, durch Vergleich mit den ersten und zweiten Referenzwertkombinationen.

8. Ermitteln des ursprünglichen Gehalts von Vitamin C aus dem ersten Ausgangsprodukt-Konzentrationsistwert und dem ersten Metabolit-Konzentrationsistwert, z.B. durch Addition dieser Werte. Berechnen des Frischegrades des Lebensmittels aus dem Verhältnis von erstem Ausgangsprodukt-Konzentrationsistwert (derzeitiger Gehalt von Vitamin C) und ursprünglichem Gehalt von Vitamin C.

9. Bewerten der Verfallsprodukte in

    a) thermischen Verfall,
    b) oxidativen Verfall,
    c) enzymatische Degradation (inklusive bakterieller Abbau),
    d) reversible Zerfallsprodukte (können im Organismus wieder in Vitamin C zurückverwandelt werden).

In diesem Beispiel zeigte es sich, dass ein Apfelsaft einen Apfelsäuregehalt von 300 mg/l Milchsäuregehalt Gehalt vom 200 mg/l aufweist, einen DHA Gehalt von 12 mg/l und einen Vitamin C Gehalt von 45 mg/l. Somit wird ein Ausgangsgehalt von 87 mg/l Vitamin C ermittelt. Der Verlust an Vitamin C und der hohe Milchsäuregehalt Gehalt zeigen, dass der Apfelsaft erhitzt wurde und bakteriell belastet ist. Es wird also ermittelt, dass der Apfelsaft nicht mehr zum Verzehr geeignet ist. Da kaum oxidativer Zerfall vorliegt, wurde der Apfelsaft wahrscheinlich nach dem Verpacken in eine Verpackung der Wärme ausgesetzt.

In einem Ausführungsbeispiel werden für eine andere Apfelsaftcharge folgende Werte mit gleicher Messmethode erhalten:

| | |
|---|---|
| erster Ausgangsprodukt-Konzentrationsistwert (Vitamin C Gehalt): | 92 mg/ml |
| zweiter Ausgangsprodukt-Konzentrationsistwert (Apfelsäuregehalt): | 500 mg/ml |
| erster Metabolit-Konzentrationsistwert (DHA Gehalt): | 13 mg/ml |
| zweiter Metabolit-Konzentrationsistwert (Milchsäuregehalt): | 20 mg/ml |

Diese Werte werden mit lebensmittelspezifischen Referenzwerten verglichen. Da der Vergleich ergibt, dass der Apfelsaft noch haltbar ist, wird nun aus dem ersten Ausgangsprodukt-Konzentrationsistwert und dem ersten Metabolit-Konzentrationsistwert ein erster Haltbarkeitsdauerwert ermittelt. Dazu wird folgender erster Verhältniswert wie folgt gebildet.

$$\frac{Konzentrationsistwert(DHA)}{Konzentrationsistwert(Vitamin\,C) + Konzentrationsistwert(DHA)} = \frac{13}{92+13} = 12,4\%$$

Somit sind 12,4 % des ursprünglichen Vitamin C zerfallen.
Es werden erste Referenzwertkombinationen bereitgestellt, die jeweils einen ersten Verhältnisreferenzwert sowie einen diesem zugeordneten ersten Haltbarkeitsdauerreferenzwert aufweisen. Jeder erste Verhältnisreferenzwert entspricht jeweils dem Verhältnis zwischen einem ersten Metabolit-Konzentrationswert und der Summe aus dem ersten Metabolit-Konzentrationswert und einem ersten Ausgangsprodukt-Konzentrationsistwert. Die ersten Referenzwertkombinationen können zum Beispiel experimentell anhand von Versuchsreihen ermittelt werden, die an entsprechenden Vergleichsapfelsäften durchgeführt werden.

Aus dem ersten Verhältniswert und den ersten Referenzwertkombinationen wird nun ein erster Haltbarkeitsdauertwert (für thermische Belastung - Vitamin C Zersetzung) bestimmt. Dieser liegt bei 190 Tagen.

Aus den zweiten Ausgangsprodukt- und Metabolit-Konzentrationsistwerten wird in entsprechender Weise ein zweiter Verhältniswert gebildet:

$$\frac{Konzentrationsistwert(Milchs\ddot{a}ure)}{Konzentrationsistwert(\ddot{A}pfels\ddot{a}ure) + Konzentrationsistwert(Milchs\ddot{a}ure)} = \frac{20}{500+20} = 3,8\%$$

Somit sind nur 3,8 % der ursprünglichen Äpfelsäure zerfallen, d.h. es liegt keine nennenswerte bakterienbedingte Zersetzung vor.

Es werden zweite Referenzwertkombinationen bereitgestellt, die jeweils einen zweiten Verhältnisreferenzwert sowie einen diesem zugeordneten zweiten Haltbarkeitsdauerreferenzwert aufweisen. Jeder zweite Verhältnisreferenzwert entspricht jeweils dem Verhältnis zwischen einem zweiten Metabolit-Konzentrationswert und der Summe aus dem zweiten Metabolit-Konzentrationswert und einem zweiten Ausgangsprodukt-Konzentrationsistwert. Die zweiten Referenzwertkombinationen können zum Beispiel experimentell anhand von Versuchsreihen ermittelt werden, die an entsprechenden Vergleichsapfelsäften durchgeführt werden.

Aus dem zweiten Verhältniswert und den zweiten Referenzwertkombinationen wird ein zweiter Haltbarkeitsdauertwert (für bakterielle Belastung - Äpfelsäurezersetzung) bestimmt. Dieser beträgt 180 Tage und entspricht dem vom Hersteller des Apfelsafts festgelegten, beispielsweise auf der Verpackung des Apfelsafts angegeben Sollwert für die Haltbarkeitsdauer. Der Sollwert für die Haltbarkeitsdauer von 180 Tagen ist also nicht überschritten und kann beibehalten werden.

Bei einem dritten Ausführungsbeispiel, bei dem eine Apfelsaft-Probe untersucht wurde, ergaben Messungen, dass 50% des ursprünglichen Vitamin C zu DHA zerfallen waren und 10% der Äpfelsäure zu Milchsäure zerfallen waren. Somit zeigte sich, dass der Haltbarkeitsdauerwert für die Vitamin C Bestimmung bei 120 Tagen liegt (d.h. 60 Tage weniger als der Sollwert, der 180 Tage beträgt) und die Bestimmung der Äpfelsäure führt durch Vergleich mit Referenzwerten zu einer Haltbarkeitsdauer aufgrund von bakterieller Zersetzung von 160 Tagen. D.h. 20 Tage weniger als der Sollwert (180 Tage).

Nun werden die ermittelten Frischeverluste miteinander verglichen, wobei festgestellt wird, dass der durch Wärmeinwirkung aufgetretene Frischeverlust (60 Tage) größer ist als der durch bakterienbedingte Zersetzung des Apfelsafts aufgetretene Frischeverlust (20 Tage).

Nun wird ein Interaktionswert für den Einfluss bereitgestellt, den der durch Wärmeinwirkung bewirkte Frischeverlust auf das Verhältnis zwischen der Konzentration von DHA und der Summe der Konzentrationen von Vitamin C und DHA hat. Da zwischen der thermischen und der bakteriellen Zersetzung eine starke Interaktion besteht, hat dieser Interaktionswert den Wert 1.

Zur Bestimmung des Gesamtfrischeverlusts wird nun der kleinere der beiden Haltbarkeitsverlustwerte mit dem Interaktionswert multipliziert und das Ergebnis dieser Multiplikation zum größeren Haltbarkeitsverlustwert addiert:

$$Gesamtfrischeverlust = 1 \times 20 \ Tage + 60 \ Tage = 80 \ Tage$$

Zur Bestimmung der Haltbarkeitsdauer des Apfelsafts wird der Gesamtfrischeverlust von dem Sollwert für die Haltbarkeitsdauer subtrahiert, was eine Haltbarkeitsdauer von 180 Tagen - 80 Tagen = 100 Tage ergibt. Somit ist der Apfelsaft statt der erwarteten 180 Tage nur noch 100 Tage haltbar.

**Patentansprüche**

1. Verfahren zur Bestimmung eines Kennwerts für die Frische und/oder den Frischeverlust einer zumindest ein erstes Ausgangsgangsprodukt enthaltenden Lebensmittel-Probe, wobei beim Auftreten einer ersten Frischeverlustart das erste Ausgangsprodukt zersetzt und mindestens ein erster Metabolit gebildet wird, wobei für die Konzentration des ersten Ausgangsprodukts ein erster Ausgangsprodukt-Konzentrationsistwert und für die Konzentration des ersten Metaboliten ein erster Metabolit-Konzentrationsistwert gemessen wird, wobei der erste Ausgangsprodukt-Konzentrationsistwert und der erste Metabolit-Konzentrationsistwert gleichzeitig gemessen werden, **dadurch gekennzeichnet, dass** die Lebensmittel-Probe ein sich von dem ersten Ausgangsgangsprodukt unterscheidendes zweites Ausgangsgangsprodukt enthält, dass beim Auftreten einer zweiten Frischeverlustart das zweite Ausgangsprodukt zersetzt und mindestens ein zweiter Metabolit gebildet wird, dass für die Konzentration des zweiten Ausgangsprodukts ein zweiter Ausgangsprodukt-Konzentrationsistwert und für die Konzentration des zweiten Metaboliten ein zweiter Metabolit-Konzentrationsistwert gemessen wird, dass die Ausgangsprodukt-Konzentrationsistwerte und die Metabolit-Konzentrationsistwerte gleichzeitig gemessen werden, dass für die erste Frischeverlustart erste Referenzwertkombinationen bereit gestellt werden, die jeweils einen vom Verhältnis zwischen dem Ausgangsprodukt-Konzentrationswert für das erste Ausgangsgangsprodukt und dem Metabolit-Konzentrationswert für den ersten Metabolit abhängigen ersten Verhältnisreferenzwert sowie einen diesem zugeordneten ersten Haltbarkeitsdauerreferenzwert aufweisen, dass ein vom Verhältnis zwischen dem ersten Ausgangsprodukt-Konzentrationsistwert und dem ersten Metabolit-Konzentrationsistwert abhängiger erster Verhältnisistwert gebildet wird, dass in Abhängigkeit von den ersten Referenzwertkombinationen und dem ersten Verhältnisistwert für die erste Frischeverlustart ein erster Haltbarkeitsdauerwert ermittelt wird, dass für die zweite Frischeverlustart zweite Referenzwertkombinationen bereit gestellt werden, die jeweils einen vom Verhältnis zwischen dem Ausgangsprodukt-Konzentrationswert für das zweite Ausgangsgangsprodukt und dem Metabolit-Konzentrationswert für den zweiten Metabolit abhängigen zweiten Verhältnisreferenzwert sowie einen diesem zugeordneten zweiten Haltbarkeitsdauerreferenzwert aufweisen, dass ein vom Verhältnis zwischen dem zweiten Ausgangsprodukt-Konzentrationsistwert und dem zweiten Metabolit-Konzentrationsistwert abhängiger zweiter Verhältnisistwert gebildet wird, dass in Abhängigkeit von den zweiten Referenzwertkombinationen und dem -zweiten Verhältnisistwert für die zweite Frischeverlustart ein zweiter Haltbarkeitsdauerwert ermittelt wird, dass ein Sollwert für die Haltbarkeitsdauer der Lebensmittel-Probe bereit gestellt wird, dass für jede Frischeverlustart jeweils ein Differenzwert für die Differenz zwischen dem für diese Frischeverlustart ermittelten Haltbarkeitsdauerwert und dem Sollwert bestimmt wird, dass ein Interaktionswert für den Einfluss, den der Frischeverlust der Frischeverlustart, für welche der größte Differenzwert ermittelt wurde, auf den Frischeverlust der anderen Frischeverlustart hat, bereitgestellt wird, und dass der Kennwert für die Frische in der Weise gebildet wird, dass alle Differenzwerte, mit Ausnahme des größten Differenzwerts, jeweils mit einem ihnen zugeordneten Interaktionswert multipliziert werden, und dass das Ergebnis dieser Multiplikation oder die Ergebnisse dieser Multiplikationen sowie der größte Differenzwert vom Sollwert für die Haltbarkeitsdauer subtrahiert werden.

2. Verfahren nach Anspruch 1, **dadurch gekennzeichnet, dass** die Lebensmittel-Probe mindestens ein drittes Ausgangsgangsprodukt enthält, das beim Auftreten einer dritten Frischeverlustart unter Bildung eines dritten Metabolits zersetzt wird, dass für die Konzentration des dritten Ausgangsprodukts ein dritter Ausgangsprodukt-Konzentrationsistwert und für die Konzentration des dritten Metaboliten ein dritter Metabolit-Konzentrationsistwert gemessen wird, und dass der Kennwert für die Frische der Lebensmittelprobe in Abhängigkeit vom Verhältnis des dritten Ausgangsprodukt-Konzentrationsistwerts zum dritten Metabolit-Konzentrationsistwert ermittelt wird.

3. Verfahren nach Anspruch 2, **dadurch gekennzeichnet, dass** für die dritte Frischeverlustart dritte Referenzwertkombinationen bereit gestellt werden, die jeweils einen vom Verhältnis zwischen dem Ausgangsprodukt-Konzentrationswert für das dritte Ausgangsgangsprodukt und dem Metabolit-Konzentrationswert für den dritten Metabolit abhängigen dritten Verhältnisreferenzwert sowie einen diesem zugeordneten dritten Haltbarkeitsdauerreferenzwert aufweisen, dass ein vom Verhältnis zwischen dem dritten Ausgangsprodukt-Konzentrationsistwert und dem dritten Metabolit-Konzentrationsistwert abhängiger dritter Verhältnisistwert gebildet wird, dass in Abhängigkeit von den dritten Referenzwertkombinationen und dem dritten Verhältnisistwert für die dritte Frischeverlustart ein dritter Haltbarkeitsdauerwert ermittelt wird, dass für jede Frischeverlustart, für die nicht der größte Differenzwert ermittelt wurde, jeweils ein Interaktionswert für den Einfluss, den der Frischeverlust der Frischeverlustart, für welche der größte Differenzwert ermittelt wurde, auf den Frischeverlust der zuerst genannten Frischeverlustart hat, bereitgestellt wird, und dass der Kennwert für die Frische aus dem Sollwert für die Haltbarkeitsdauer, den Differenzwerten und den Interaktionswerten gebildet wird.

4. Verfahren nach einem der Ansprüche 1 bis 3, **dadurch gekennzeichnet, dass** die Ausgangsprodukt-Konzentrationsistwerte und die Metabolit-Konzentrationsistwerte mittels Fourier-Transfbrmaflons-Infrarotspektroskopie gemessen werden.

5. Verfahren nach Anspruch 4, **dadurch gekennzeichnet, dass** zum Ermitteln der Metabolit-Konzentrationsistwerte und der Ausgangsprodukt-Konzentrationsistwerte Messwerte erfasst werden, dass die Messwerte matrixspezifisch korrigiert werden, und dass die Konzentrationsistwerte mit Hilfe der korrigierten Messwerte ermittelt werden.

6. Verfahren nach einem der Ansprüche 1 bis 5, **dadurch gekennzeichnet, dass** eine Art des Frischeverlustes die Autolyse ist.

7. Verfahren nach einem der Ansprüche 1 bis 6, **dadurch gekennzeichnet, dass** eine Art des Frischeverlustes ein thermischer Frischeverlust, ein oxidativer Frischeverlust, ein bakterieller Frischeverlust und/oder ein photochemischer Frischeverlust ist.

8. Verfahren nach Anspruch 1 bis 7, **dadurch gekennzeichnet, dass** gleichzeitig zur Messung der Ausgangsprodukt-Konzentrationsistwerte und zur Messung der Metabolit-Konzentrationsistwerte der Nährwert der Probe bestimmt wird.

9. Verfahren nach einem der Ansprüche 1 bis 8, **dadurch gekennzeichnet, dass** das erste Ausgangsgangsprodukt Ascorbinsäure, ein erster erster Metabolit Dehydroascorbinsäure, ein zweiter erster Metabolit Diketogulonsäure und ein dritter erster Metabolit Hydoxymethylfurfural ist und wobei das zweite Ausgangsgangsprodukt Milchsäure und der zweite Metabolit Glucose ist.

10. Verfahren nach Anspruch 1, **dadurch gekennzeichnet, dass** das erste Ausgangsgangsprodukt Vitamin C, der erste Metabolit Dehydroascorbinsäure, das zweite Ausgangsgangsprodukt Äpfelsaure und der zweite Metabolit Milchsäure ist.

**Claims**

1. Method for determining a characteristic value for the freshness and/or the loss of freshness of a food sample containing at least one first starting product, wherein the first starting product decomposes and at least one first metabolite is formed upon the occurrence of a first type of loss of freshness, wherein a first actual starting-product concentration value is measured for the concentration of the first starting product and a first actual metabolite concentration value is measured for the concentration of the first metabolite, wherein the first actual starting-product concentration value and the first actual metabolite concentration value are measured simultaneously, **characterized in that** the food sample contains a second starting product different from the first starting product, **in that** the second starting product decomposes and at least one second metabolite is formed upon the occurrence of a second type of loss of freshness, **in that** a second actual starting-product concentration value is measured for the concentration of the second starting product and a second actual metabolite concentration value is measured for the concentration of the second metabolite, **in that** the actual starting-product concentration values and the actual metabolite concentration values are measured simultaneously, **in that** the first type of loss of freshness is provided with first reference value combinations which have in each case a first reference ratio value dependent on the ratio between the starting-product concentration value for the first starting product and the metabolite concentration value for the first metabolite as well as a first reference shelf-life value assigned thereto, **in that** a first actual ratio value dependent on the ratio between the first actual starting-product concentration value and the first actual metabolite concentration value is formed, **in that** a first shelf-life value is ascertained depending on the first reference value combinations and the first actual ratio value for the first type of loss of freshness, **in that** the second type of loss of freshness is provided with second reference value combinations which have in each case a second reference ratio value dependent on the ratio between the starting-product concentration value for the second starting product and the metabolite concentration value for the second metabolite as well as a second reference shelf-life value assigned thereto, **in that** a second actual ratio value dependent on the ratio between the second actual starting-product concentration value and the second actual metabolite concentration value is formed, **in that** a second shelf-life value is ascertained depending on the second reference value combinations and the second actual ratio value for the second type of loss of freshness, **in that** a nominal value for the shelf life of the food sample is provided, **in that** each type of loss of freshness has determined therefor a difference value for the difference between the shelf-life value ascertained for said type of loss of freshness and the nominal value, **in that** an interaction value for the influence which the loss of freshness of the type of loss of freshness for which the largest difference value has been ascertained has on the loss of freshness of the other type of loss of freshness is provided, and **in that** the characteristic value for freshness is formed in such a way that all difference values, with the exception of the largest difference value, are each multiplied by an interaction value assigned thereto, and **in that** the result of this multiplication or the results of these multiplications as well as the largest difference value are subtracted from the nominal value for shelf life.

2. Method according to Claim 1, **characterized in that** the food sample contains at least one third starting product which is decomposed upon the occurrence of a third type of loss of freshness to form a third metabolite, **in that** a third actual starting-product concentration value is measured for the concentration of the third starting product and a third actual metabolite concentration value is measured for the concentration of the third metabolite, and **in that** the characteristic value for the freshness of the food sample is ascertained depending on the ratio of the third actual starting-product concentration value to the third actual metabolite concentration value.

**3.** Method according to Claim 2, **characterized in that** the third type of loss of freshness is provided with third reference value combinations which have in each case a third reference ratio value dependent on the ratio between the starting-product concentration value for the third starting product and the metabolite concentration value for the third metabolite as well as a third reference shelf-life value assigned thereto, **in that** a third actual ratio value dependent on the ratio between the third actual starting-product concentration value and the third actual metabolite concentration value is formed, **in that** a third shelf-life value is ascertained depending on the third reference value combinations and the third actual ratio value for the third type of loss of freshness, **in that** each type of loss of freshness for which the largest difference value has not been ascertained is provided with an interaction value for the influence which the loss of freshness of the type of loss of freshness for which the largest difference value has been ascertained has on the loss of freshness of the first-mentioned type of loss of freshness, and **in that** the characteristic value for freshness is formed from the nominal value for shelf life, from the difference values and from the interaction values.

**4.** Method according to any of Claims 1 to 3, **characterized in that** the actual starting-product concentration values and the actual metabolite concentration values are measured by means of Fourier-transform infrared spectroscopy.

**5.** Method according to Claim 4, **characterized in that** measurement values are captured in order to ascertain the actual metabolite concentration values and the actual starting-product concentration values, **in that** the measurement values are corrected in a matrix-specific manner, and **in that** the actual concentration values are ascertained with the aid of the corrected measurement values.

**6.** Method according to any of Claims 1 to 5, **characterized in that** one type of loss of freshness is autolysis.

**7.** Method according to any of Claims 1 to 6, **characterized in that** one type of loss of freshness is a thermal loss of freshness, an oxidative loss of freshness, a bacterial loss of freshness and/or a photochemical loss of freshness.

**8.** Method according to Claims 1 to 7, **characterized in that** the nutritional value of the sample is determined at the same time as the measurement of the actual starting-product concentration values and the measurement of the actual metabolite concentration values.

**9.** Method according to any of Claims 1 to 8, **characterized in that** the first starting product is ascorbic acid, a first first metabolite is dehydroascorbic acid, a second first metabolite is diketogulonic acid and a third first metabolite is hydroxymethylfurfural, and wherein the second starting product is lactic acid and the second metabolite is glucose.

**10.** Method according to Claim 1, **characterized in that** the first starting product is vitamin C, the first metabolite is dehydroascorbic acid, the second starting product is malic acid and the second metabolite is lactic acid.

**Revendications**

**1.** Procédé de détermination d'une valeur caractéristique pour la fraîcheur et/ou la perte de fraîcheur d'un échantillon de produit alimentaire contenant au moins un premier produit de départ, le premier produit de départ étant décomposé lors de l'occurrence d'un premier type de perte de fraîcheur et au moins un premier métabolite étant formé, une première valeur réelle de concentration de produit de départ étant mesurée pour la concentration du premier produit de départ et une première valeur réelle de concentration de métabolite étant mesurée pour la concentration du premier métabolite, la première valeur réelle de concentration de produit de départ et la première valeur réelle de concentration de métabolite étant mesurées simultanément, **caractérisé en ce que** l'échantillon de produit alimentaire contient un deuxième produit de départ différent du premier produit de départ, **en ce que** lors de l'occurrence d'un deuxième type de perte de fraîcheur, le deuxième produit de départ est décomposé et au moins un deuxième métabolite est formé, **en ce qu'**une deuxième valeur réelle de concentration de produit de départ est mesurée pour la concentration du deuxième produit de départ et une deuxième valeur réelle de concentration de métabolite est mesurée pour la concentration du deuxième métabolite, **en ce que** les valeurs réelles de concentration de produit de départ et les valeurs réelles de concentration de métabolite sont mesurées simultanément, **en ce que** des premières combinaisons de valeurs de référence sont mises à disposition pour le premier type de perte de fraîcheur, qui comprennent chacune une première valeur de référence de rapport dépendante du rapport entre la valeur de concentration de produit de départ pour le premier produit de départ et la valeur de concentration de métabolite pour le premier métabolite, ainsi qu'une première valeur de référence de durée de stabilité attribuée à celle-ci, **en ce qu'**une première valeur réelle de rapport dépendante du rapport entre la première valeur réelle de concentration de produit de départ et la première valeur réelle de concentration de métabolite est formée, **en ce qu'**une première valeur de durée de stabilité est déterminée en fonction des premières combinaisons de valeurs de référence et de la première valeur réelle de rapport pour le premier type de perte de fraîcheur, **en ce que** des deuxièmes combinaisons de valeurs de référence sont mises à disposition pour le deuxième type de perte de fraîcheur, qui comprennent chacune une deuxième valeur de référence de rapport dépendante du rapport entre la valeur de concentration de produit de départ pour le deuxième produit de départ et la valeur de concentration de métabolite pour le deuxième métabolite, ainsi qu'une deuxième valeur de référence de durée de stabilité attribuée à celle-ci, **en ce qu'**une deuxième valeur réelle de rapport dépendante du rapport entre la deuxième valeur réelle de concentration de produit de départ et la deuxième valeur réelle de concentration de métabolite est formée, **en ce qu'**une deuxième valeur de durée de stabilité est déterminée en fonction des deuxièmes combinaisons de valeurs de référence et la deuxième valeur réelle de rapport pour le deuxième type de perte de fraîcheur, **en ce qu'**une valeur de consigne pour la durée de stabilité de l'échantillon de produit alimentaire est mise à disposition, **en ce qu'**une valeur différentielle pour la différence entre la valeur de durée de stabilité calculée pour ce type de perte de fraîcheur et la valeur de consigne est déterminée pour chaque type de perte de fraîcheur, **en ce qu'**une valeur d'interaction pour l'effet que la perte de fraîcheur du type de perte de fraîcheur pour lequel la plus grande valeur différentielle a été déterminée a sur la perte de fraîcheur de l'autre type de perte de fraîcheur est mise à disposition, et **en ce que** la valeur caractéristique pour la fraîcheur est formée par multiplication de toutes les valeurs différentielles, à l'exception de la plus grande valeur différentielle, avec une valeur d'interaction attribuée à chacune d'entre elles, et soustraction du résultat de cette multiplication ou des résultats de ces multiplications, ainsi que de la plus grande valeur différentielle, de la valeur de consigne pour la durée de stabilité.

2. Procédé selon la revendication 1, **caractérisé en ce que** l'échantillon de produit alimentaire contient au moins un troisième produit de départ, qui est décomposé lors de l'occurrence d'un troisième type de perte de fraîcheur pour former un troisième métabolite, **en ce qu'**une troisième valeur réelle de concentration de produit de départ est mesurée pour la concentration du troisième produit de départ et une troisième valeur réelle de concentration de métabolite est mesurée pour la concentration du troisième métabolite, et **en ce que** la valeur caractéristique pour la fraîcheur de l'échantillon de produit alimentaire est déterminée en fonction du rapport entre la troisième valeur réelle de concentration de produit de départ et la troisième valeur réelle de concentration de métabolite.

3. Procédé selon la revendication 2, **caractérisé en ce que** des troisièmes combinaisons de valeurs de référence sont mises à disposition pour le troisième type de perte de fraîcheur, qui comprennent chacune une troisième valeur de référence de rapport dépendante du rapport entre la valeur de concentration de produit de départ pour le troisième produit de départ et la valeur de concentration de métabolite pour le troisième métabolite, ainsi qu'une troisième valeur de référence de durée de stabilité attribuée à celle-ci, **en ce qu'**une troisième valeur réelle de rapport dépendante du rapport entre la troisième valeur réelle de concentration de produit de départ et la troisième valeur réelle de concentration de métabolite est formée, **en ce qu'**une troisième valeur de durée de stabilité est déterminée en fonction des troisièmes combinaisons de valeurs de référence et de la troisième valeur réelle de rapport pour le troisième type de perte de fraîcheur, **en ce qu'**une valeur d'interaction est mise à disposition pour chaque type de perte de fraîcheur pour lequel la plus grande valeur différentielle n'a pas été déterminée, pour l'effet que la perte de fraîcheur du type de perte de fraîcheur pour lequel la plus grande valeur différentielle a été déterminée a sur la perte de fraîcheur du type de perte de fraîcheur mentionné en premier, et **en ce que** la valeur caractéristique pour la fraîcheur est formée à partir de la valeur de consigne pour la durée de stabilité, des valeurs différentielles et des valeurs d'interaction.

4. Procédé selon l'une quelconque des revendications 1 à 3, **caractérisé en ce que** les valeurs réelles de concentration de produit de départ et les valeurs réelles de concentration de métabolite sont mesurées par spectroscopie infrarouge à transformée de Fourier.

5. Procédé selon la revendication 4, **caractérisé en ce que** des valeurs de mesure sont enregistrées pour déterminer les valeurs réelles de concentration de métabolite et les valeurs réelles de concentration de produit de départ, **en ce que** les valeurs de mesure sont corrigées d'une manière spécifique à la matrice, et **en ce que** les valeurs réelles de concentration sont déterminées à l'aide des valeurs de mesure corrigées.

6. Procédé selon l'une quelconque des revendications 1 à 5, **caractérisé en ce qu'**un type de perte de fraîcheur est l'autolyse.

7. Procédé selon l'une quelconque des revendications 1 à 6, **caractérisé en ce qu'**un type de perte de fraîcheur est une perte de fraîcheur thermique, une perte de fraîcheur oxydative, une perte de fraîcheur bactérienne et/ou une perte de fraîcheur photochimique.

8. Procédé selon les revendications 1 à 7, **caractérisé en ce que**, simultanément à la mesure des valeurs réelles de concentration de produit de départ et à la mesure des valeurs réelles de concentration de métabolite, la valeur nutritive de l'échantillon est déterminée.

9. Procédé selon l'une quelconque des revendications 1 à 8, **caractérisé en ce que** le premier produit de départ est l'acide ascorbique, un premier premier métabolite est l'acide déshydroascorbique, un deuxième premier métabolite est l'acide dicétogulonique et un troisième premier métabolite est l'hydroxyméthylfurfural, et le deuxième produit de départ est l'acide lactique et le deuxième métabolite est le glucose.

10. Procédé selon la revendication 1, **caractérisé en ce que** le premier produit de départ est la vitamine C, le premier métabolite est l'acide déshydroascorbique, le deuxième produit de départ est l'acide malique et le deuxième métabolite est l'acide lactique.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **OLGA SOLOMON et al.** Effect of oxygen and fluorescent light on the quality of orange juice during storage at 8°C. *Food Chemistry,* 01. Januar 1995, vol. 53 (4), ISSN 0308-8146, 363-368

- Studies on the quality evaluation of canned marine products - I. Determination of the ratio of dimethylamine to trimethylamine in canned albacore. **TOSHIO TOKUNAGA.** Nippon Suisan Gakkaishi - Bulletin Of The Japanes Society Of Scientific Fisheries. Nippon Suisan Gakkai, 01. Januar 1975, vol. 41, 547-553